# EUROPEAN PATENT APPLICATION

(11) **EP 4 667 006 A2**
(43) Date of publication of application: **24.12.2025**
(21) Application number: 25214459.7
(22) Date of filing: 29.05.2020
(51) Int. Cl.: A61K 31/506

(54) **TALTIRELIN USE**

(30) Priority: 29.05.2019 GB 201907591
(62) Divisional of application: 20730699.4
(71) Applicant: Eolas Research Limited, Edinburgh EH9 3AL (GB)
(72) Inventor: FORBES, Iain, Edinburgh, EH9 3AL (GB); FORBES, William, Edinburgh, EH9 3AL (GB)
(74) Representative: Jacob, Reuben Ellis

(57) **Abstract**

Use of a composition for the prevention, amelioration and/ or treatment of a condition associated with cell or tissue senescence and/ or inflammation and/ or autophagy, the composition comprising an active ingredient taltirelin or an analogue thereof.

## Description

The invention relates to the use of a composition for the prevention, amelioration and/ or treatment of conditions associated with ageing and senescence, the process of ageing, premature ageing and susceptibility to disease as a result of ageing and/ or senescence, and/ or inflammatory and/ or autoimmune conditions.

Thyrotropin-releasing hormone (TRH) is a small peptide which is found in the hypothalamus of the brain and emanates from a group of neurones. The native peptide is highly bio-active and controls the release of thyroglobulin, which in turn is responsible for the induction of thyroid hormones. TRH and associated analogues are useful for the treatment or prophylaxis of central nervous disorders (e.g. as described in JPS 62234029 and EP 0168042).

Taltirelin (also known as Ceredist) is a thyrotropin-releasing hormone analogue which mimics the physiological actions of TRH but has a much longer half life and duration of effects. Taltirelin has been used in the treatment of neurological conditions.

Ageing in mammals involves the progressive loss of function in cells and/or tissues involving a number of interconnected biological processes including, cell senescence, inflammaging/ immunosenescence, autophagy, reduction in the stem cell pool, reduction in stem cell regeneration and mitochondrial dysfunction. This age-related loss of function is influenced by both genetics and environmental factors and leads to a wide range of degenerative conditions, diseases and pathologies. Ageing is the greatest risk factor for many of the most common morbidities, including cancer, cardiovascular and neurodegenerative diseases as well as a reduction in immunity resulting in reduced T-Cell and B-Cell production and diminished function of lymphocytes leading to a diminished response to viral, bacterial and parasitic infections.

The term senescence, including cellular senescence, involves perceptible changes in cells such as telomere attrition, double-strand DNA breaks, persistent DNA damage response, oxidative stress, oncogene activation and an increase in the levels of cell-cycle arrest proteins p16 INK4a and p21 all leading to an exit from the normal cell-cycle, a process termed senescence associated growth arrest. These senescent cells, with age, tend to accumulate and become increasingly harmful to surrounding cells and tissues and in addition become increasingly likely to escape from the state of growth arrest to become cancerous. Further, these accumulating senescent cells result in an increasing loss in the physiological functioning in a tissue and the prevention of new healthy cells from replacing them. Senescent cells in the long term contribute further to ageing effects by secreting pro-inflammatory and growth factors. The process of senescence may be natural, premature, induced or accelerated. Induced or accelerated senescence has been found to be involved in some diseases, and in transplanted cells, tissues and organs. Accelerated senescence has been observed in diseased organs such as the kidney.

The term inflammaging involves chronic low grade inflammatory processes, in the absence of infection, linked to ageing *via* immunosenescence. Senescent cells develop a so-called senescence-associated secretory phenotype (SASP), which involves the secretion of pro-inflammatory factors which are thought to contribute to inflammaging. This SASP is a major risk factor for age-related diseases.

The term autophagy, which includes mitophagy, is involved the intracellular degradation process in healthy cells that helps maintain cellular homeostasis by degrading and recycling dysfunctional and excess cellular components. Many physiological functions are regulated by autophagy, including senescence, longevity tissue remodelling and cell survival during stress. Reduced autophagy has been found to be involved in the pathogenesis and/ or progression of age-related diseases such as inflammatory and autoimmune diseases including osteoarthritis, lupus erythematosus, Crohn's disease, and inflammatory bowel disease. In addition, reduced autophagy has also been associated with cancers, metabolic diseases, fibrotic diseases including for example idiopathic pulmonary disease, heart disease, as well as neurodegenerative diseases such as Parkinson's disease and Huntington's disease. Reduced autophagy has also been linked to psychiatric disorders such as schizophrenia and bipolar disorder.

The terms stem cell pool and stem cell regeneration involve the population of cells that can replenish tissue with a range of newly differentiated cell types and the regeneration of a variety of cell types from so-called stem cells, thus allowing the repair and maintenance of healthy tissue of tissues. Ageing is characterised by a reduction in the capability of stem cells to regenerate cells in tissues.

The present invention seeks to provide a composition for use in preventing, ameliorating and/ or treating conditions which involve the processes of ageing, including senescence and/ or inflammation and/ or autophagy and/ or reduction in the stem cell pool and/ or stem cell regenerative capability including lymphoid biased stem cells.

According to the invention, there is provided use of a composition for the prevention, amelioration and/ or treatment of a condition associated with cell or tissue senescence and/ or inflammation and/ or autophagy, the composition comprising an active ingredient taltirelin or an analogue thereof.

Preferably, the composition comprises the active ingredient taltirelin having the name N-[[(4S)-Hexahydro-1-methyl-2,6-dioxo-4-pyrimidinyl]carbonyl]-L-histidyl-L-prolinamide.

Preferably the composition comprises the active ingredient taltirelin having the structure shown in (1) below: 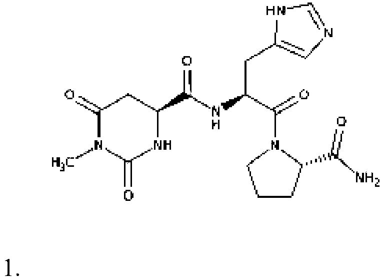 or an analogue thereof.

Preferably, the composition may comprise at least one acceptable carrier and/ or may be carried within a vesicle, micelle, liposome, nanoparticle or other suitable vehicle.

Preferably, the composition may be used at a dose of around 5 to 15 mg per kilogram of body weight per day, especially at a dose of 10 mg per kilogram of body weight per day. Typically, administration may be via an oral or parenteral (e.g. via the intravenous, intramuscular or subcutaneous) route. Typically, the therapeutic dose of the composition depends on the route of administration; the age, weight and condition of patients; and the particular disease to be treated.

Preferably, the composition of the present invention comprises anti-senescence, and/or anti-inflammatory and/ or anti-proliferative and/ or pro-autophagic and/ or pro- stem cell regenerative properties and/ or stem cell pool maintenance properties. Typically, the use of the composition of the present invention is involved in the induction of FOXO gene expression, which expression decreases with age and which increased expression advantageously reduces the effects of ageing and increases longevity, increases immune cell homeostasis, differentiation, reactivation and self-renewal, controls the trajectory of T-Cell dependent immune response, increases survival of CD8+ memory cells and antiviral CD8+ cells and in addition advantageously activates and regulates key leukocytes, including the formation of T-regulatory cells and B-lymphocytes needed to maintain homeostasis and respond to infection in mucosal tissues including oral mucosal tissues.

Preferably, the use of the composition is for the prevention, amelioration and/ or treatment of a condition caused by an infective agent. Preferably, the use of the composition is for the prevention, amelioration and/ or treatment of a condition caused by a virus. Preferably, the use of the composition is for the prevention, amelioration and/ or treatment of a condition caused by a coronavirus. Preferably, the use of the composition is for the prevention, amelioration and/ or treatment of a condition caused by severe acute respiratory syndrome coronavirus 2 (SARS-CoV-2).

Preferably, the use of the composition augments the immune response, induces the clonal expansion of T-cell and B-cell populations, increases the reactivation of T-cells and/ or increases the persistence of immune T-cell memory via the upregulation and/ or enhanced survival of CD8+ cells.

Preferably, the use of the composition controls T-cell trafficking to other tissues and/ or regulates the homing of peripheral B-cells.

Preferably, the use of the composition is for the prevention, amelioration and/ or treatment of a condition associated with oxidative stress, in particular in relation autoimmune diseases, diseases that are stress mediated and in infection, including viral infection, including SARS-CoV-2.

Preferably, the use of the composition decreases fibrin deposition in the lungs to ameliorate lung pathology during and/ or after viral infection and/ or to inhibit the activation of pathogenic Th17 cells to ameliorate their effect on lung pathology.

Preferably, the use of the composition is to block the activation of the TMPRSS2 receptor on host cells before and during a viral infection and/ or to prevent cleavage of the spike protein of a coronavirus, particularly of SARS-CoV-2.

Preferably, the use of the composition increases the innate immune response, including reducing susceptibility to viral infection, viral entry to patients and/ or viral replication.

Preferably, the use of the composition of the invention is for the prevention, amelioration and/ or treatment of an inflammatory and/ or autoimmune condition. Preferably, the composition is for the prevention, amelioration and/ or treatment of ulcerative colitis and/ or multiple sclerosis.

Preferably, the use of the composition of the present invention is for the prevention, amelioration and/ or treatment of a condition associated with fibrosis. Typically, the use of the composition is for the prevention, amelioration and/ or treatment of a condition associated with fibrosis, including age related fibrosis.

Typically, the use of the composition of the present invention is for the prevention, amelioration and/ or treatment of idiopathic renal fibrosis, fibrosis in transplanted tissues and organs, idiopathic pulmonary fibrosis (the basis of chronic obstructive pulmonary disease, i.e. COPD) and/ or cystic fibrosis.

Preferably, the use of the composition of the present invention is for the prevention, amelioration and/ or treatment of a condition wherein fibrosis affects the subcutaneous fat of an individual. Typically, the use of the composition is for the prevention of a condition resulting in the formation of cellulite. Typically, cellulite is formed as a result of a fibrosis process that affects subcutaneous fat in an individual.

Typically, the composition of the present invention is involved in inhibiting two processes that are involved in fibrosis, wherein the processes are the Fibroblast to Myofibroblast Transition (FMT) and the Epithelial to Mesenchymal Transition (EMT).

Preferably, the use of the composition of the present invention is for the prevention, amelioration and/ or treatment of a condition associated with reduced cellular autophagy. Typically, the use of the composition is for the prevention, amelioration and/ or treatment of a condition associated with age related reduction in autophagy.

Preferably, the use of the composition of the present invention is for the prevention, amelioration and/ or treatment of a condition associated with a reduction in stem cell pool maintenance and/ or stem cell regenerative capability. Typically, the use of the composition is for the prevention, amelioration and/ or treatment of a condition associated with age related reduction in the stem cell pool and/ or stem cell regenerative capability including lymphoid biased stem cells.

Preferably, the use of the composition of the present invention is for the prevention, amelioration and/ or treatment of a condition associated with metabolic syndrome. Advantageously, the composition of the present invention suppresses adipogenesis and/ or regulates glucose metabolism.

Preferably, the use of the composition of the present invention is for the prevention, amelioration and/ or treatment of a condition associated with obesity, type 2 diabetes and/ or associated disorders.

Preferably, the use of the composition of the present invention is for the prevention, amelioration and/ or treatment of a condition associated with a cancer, preferably a drug resistant cancer. Preferably, the use of the composition of the present invention is for the prevention, amelioration and/ or treatment of a condition associated with epithelial to mesenchymal cancer (i.e. EMT cancer). Advantageously, the composition of the present invention affects the expression of key genes involved in the development of certain cancers, where there is an epithelial to mesenechymal cell transition (EMT). Preferably, the use of the composition of the present invention is for the prevention, amelioration and/ or treatment of an epithelial cancer and/ or bone cancer.

Preferably, the use of the composition of the present invention is for the prevention, amelioration and/ or treatment of cancer, such as non small cell lung carcinoma, hepatocellular carcinoma, pancreatic ductal adenocarcinma and/ or glioblastoma. Preferably, the composition of the present invention has anti-proliferative and/ or anti-cancer properties. Advantageously, the composition readily penetrates the blood brain barrier of a patient, thus facilitating treatment of brain primary cancers and metastases.

Preferably, the use of the composition of the present invention is for the prevention, amelioration and/ or treatment of a bone related disease. Preferably, the use of the composition of the present invention is for the prevention, amelioration and/ or treatment of osteoarthritis, periodontitis, invertebral disk degeneration, osteosarcoma, osteoporosis and/ or cleidocranial dysplasia syndrome.

Preferably, the use of the composition of the present invention is for the prevention, amelioration and/ or treatment of a skin condition. Preferably, the use of the composition of the present invention is for the prevention, amelioration and/ or treatment of acne, psoriasis, eczema and/ or vitiligo.

Preferably, the use of the composition of the present invention is to modulate and/ or counter the side effects of glucocorticoids. One of the problems associated with the use of glucocorticoids is that these steroids, while having good anti-inflammatory action, typically have side effects during long term use. Such side effects may include the induction of osteoporotic processes, skin thinning, etc. Typically, the use of the composition of the present invention induces anti-inflammatory effects that increase gene expression of a gene involved in osteogenesis. Typically, the same gene may be silenced by glucocorticoids. Advantageously, the composition of the present invention may be used in parallel with glucocorticoids to reduce the risk of osteoporosis, skin thinning, etc.

In one embodiment, the composition may be used as a clinical dermal product. In one embodiment, the composition may be applied to the hair and/ or scalp. Typically, the composition may be provided in the form of a serum, lotion, cream, gel, powder, ointment or other medium suitable for the topical administration of the composition to the skin, or for administration to the hair and/ or scalp.

In one embodiment, the composition of the present invention may be used for the prevention, amelioration and/ or treatment of malaria.

In one embodiment, the composition of the present invention may be used for the promotion of longevity (extended life).

In one embodiment, the composition of the present invention may be used for the prevention, amelioration and/ or treatment of periodontitis and infection of oral mucosal tissue.

In one embodiment, the composition of the present invention may be used for the prevention, amelioration and/ or treatment of viral entry to and subsequent replication in tissues exposed to the external environment including the nasal passages. Typically, the composition may be provided in the form of a serum, lotion, cream, gel, ointment or other medium suitable for the topical administration of the composition to the nasal passages or other tissues exposed to the external environment.

In one embodiment, the composition may comprise a pharmaceutically acceptable carrier or delivery system which may be in the form of water and oil emulsions, suspensions, colloids, microemulsions, suspensions or emulsions of vesicles, micelles, liposomes, microparticles, nanoparticles, powders or anhydrous compositions. The composition may also be coated or combined with a material such as a lipid or sugar, or a combination thereof. In one embodiment, the composition may be linked to a protein, amino acid, aryl group, alkyl group, fatty acid, sugar, lipid, flavonoid, sugar, salt or ester or other functional group, or an isoform thereof. Preferably, the coating or target-guiding material may dissolve to allow efficient and sufficient delivery of the composition to the cells, tissues or organs.

In one embodiment, the composition may be administered by mouth or by a parenteral route. Typically, the composition may be administered by a dermal, intradermal, transdermal, topical, intramuscular, subcutaneous, intravenous, nasal, oral, sublingual, lingual or rectal route, or by inhalation or instillation. In another embodiment, the composition may be administered by incorporation in an implant, or by use of a pumping device. In one embodiment, the composition may be administered in an infusion which is circulated through organs, or added to cells, tissues or isolated organs. In one embodiment, the composition may be administered in the form of a tablet.

In one embodiment, the composition may be administered in combination with an adjuvant, such as an anti-inflammatory drug, an extract or a natural agent. In another embodiment, the composition may further comprise an antioxidant, and/ or inhibitor of Nuclear Factor-kappaB (NF-kappa B), or an inhibitor of a matrix metalloproteinase (MMP), or an antibiotic or vitamin, for example, vitamin D, vitamin D3, vitamin C dehydroascorbate, vitamin B, vitamin E, or other suitable vitamin and/ or co-factor.

In one embodiment, the composition may be incorporated within a food or drink.

In one embodiment, the composition may be manufactured in a laboratory or on an industrial scale by solid-phase synthesis or other means, and may be purified to provide a therapeutically effective dose of the composition.

Advantageously, the composition may be used in human therapeutics and/ or in veterinary practice, and is typically administered to a mammalian subject.

Advantageously, the composition may be administered with a high degree of safety.

The term senescence may relate to natural, premature, induced or accelerated senescence.

According to another aspect of the invention, there is provided use of a composition for the prevention, amelioration and/ or treatment of a condition associated with a virus, a condition associated with a coronavirus, a condition caused by severe acute respiratory syndrome coronavirus 2 (SARS-CoV-2), fibrosis such as idiopathic renal fibrosis, fibrosis in transplanted tissues and organs, idiopathic pulmonary fibrosis (the basis of chronic obstructive pulmonary disease, i.e. COPD), cystic fibrosis; a condition associated with reduced cellular autophagy; a condition associated with a reduction in stem cell pool maintenance and/ or stem cell regenerative capability; a condition associated with metabolic syndrome; a condition associated with obesity, type 2 diabetes and associated disorders; a condition associated with cancer, such as a drug resistant cancer, epithelial to mesenchymal (EMT) cancer, epithelial cancer, bone cancer, non small cell lung carcinoma, hepatocellular carcinoma, pancreatic ductal adenocarcinoma, glioblastoma, brain primary cancer and metastases; a bone related condition such as osteoarthritis, periodontitis, invertebral disk degeneration, osteosarcoma, osteoporosis, cleidocranial dysplasia syndrome; a skin condition such as acne, psoriasis, eczema, vitiligo; and/ or malaria, the composition comprising an active ingredient taltirelin or an analogue thereof.

Preferably, the composition comprises taltirelin having the name (N-[[(4S)-Hexahydro-1-methyl-2,6-dioxo-4-pyrimidinyl]carbonyl]-L-histidyl-L-prolinamide).

Preferably, the composition comprises taltirelin having the structure shown in (1) below:

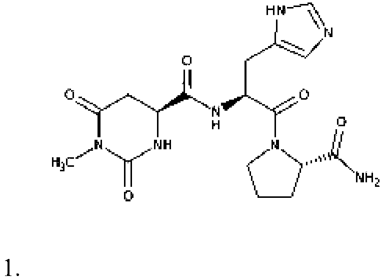

Variations and modifications of the invention are included within the scope of the invention and will be understood by those skilled in the art.

The invention will be further described by way of example and with reference to the following figures, in which:
Figure 1 is a graph illustrating the increased expression of FOXO1 and FOXO3 genes in fibroblasts by the administration of taltirelin; and
Figure 2 is a graph illustrating the increased expression of EFNB1, NFE2L2, SERPINE1 and SIRT1 genes in fibroblasts by administration of taltirelin.

With reference to the figures, there is provided use of a composition for the prevention, amelioration and/ or treatment of a condition associated with cell or tissue senescence and/ or inflammation and/ or autophagy, the composition comprising an active ingredient taltirelin (N-[[(4S)-Hexahydro-1-methyl-2,6-dioxo-4-pyrimidinyl]carbonyl]-L-histidyl-L-prolinamide) having the structure: 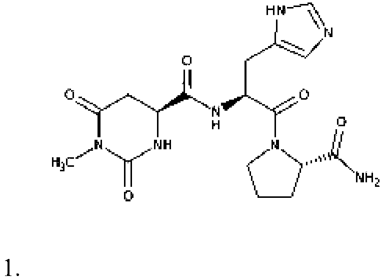 or an analogue thereof.

The composition may comprise at least one acceptable carrier and/ or may be carried within a vesicle, micelle, liposome, nanoparticle or other suitable vehicle.

The composition may be used at a dose of around 5 to 15 mg per kilogram of body weight per day, especially at a dose of 10 mg per kilogram of body weight per day. Typically, administration may be via an oral or parenteral (e.g. via the intravenous, intramuscular or subcutaneous) route. Typically, the therapeutic dose of the composition depends on the route of administration; the age, weight and condition of patients; and the particular disease to be treated.

The composition of the present invention comprises anti-senescence, and/or anti-inflammatory and/ or anti-proliferative and/ or pro-autophagic and/ or pro- stem cell regenerative properties and/ or stem cell pool maintenance properties. The use of the composition of the present invention is involved in the induction of FOXO gene expression, which expression advantageously reduces the effects of ageing and increases longevity.

### Description of FOXO gene effects:

The FOXO transcription factors are encoded by the FOXO genes and in mammals comprise FOXO1, FOXO3, FOXO4 and FOXO6.

FOXO1 is involved in the regulation of proliferation, oxidative stress resistance, metabolism, inflammation, ageing, activation of autophagy and apoptosis. The FOXO1 gene regulates glucose levels in gluconeogenesis and is a negative regulator of adipogenesis and also regulates glycogenolysis. FOXO1 typically plays a role as a tumour suppressor and may induce apoptosis is some cancers and also suppress the metastasis of some cancer cell types. The transcription factor of the FOXO1 gene (Foxo1) is decreased in certain drug resistant cancer cells. The transcription factor Foxo1 also regulates osteoblast differentiation and skeletogenesis and therefore is of importance in disease of bone. Furthermore, FOXO1 is a potent inhibitor of fibrogenic signaling and extra-cellular matrix deposition which is crucial in the pathogenesis and progression of fibrosis. Foxo1 transcription factor represses the Epithelial - Mesenchymal transition (EMT) which is involved in the pathogenesis of fibrosis and in (EMT) cancers. Foxo1, through its induction of autophagy may influence adult neurogenesis by overcoming aberrant dendritic morphology, particularly in the subgranular zone of the hippocampal nucleus and also in the subventricular zone - areas involved in memory, learning and cognitive function.

FOXO3 is involved the regulation of proliferation, oxidative stress resistance, metabolism, cellular differentiation, inflammation, ageing, proteostasis, stem cell pool maintenance, activation of autophagy and apoptosis.

Genetic polymorphisms of FOXO3 have shown consistent associations with human longevity and in *in-vivo* models.

FOXO3 enhances cell self-renewal via the activation of Notch signalling and has been shown to enhance pluripotency in some stem cells. FOXO3 is crucial for the regulation of adult stem cell homeostasis and is the primary driver of the maintenance of the stem cell pool in mammals. FOXO3 has also been shown to promote the self re-renewal of stem cells during muscle regeneration. Foxo3, through its induction of autophagy may influence adult neurogenesis by overcoming aberrant dendritic morphology, particularly in the subgranular zone of the hippocampal nucleus and also in the subventricular zone, i.e. areas involved in memory, learning and cognitive function.

The transcription factor of the FOXO3 gene (Foxo3) is decreased in certain drug resistant cancer cells.

Furthermore, FOXO3 is a potent inhibitor of fibrogenic signalling and extra-cellular matrix deposition that are crucial in the pathogenesis and progression of fibrosis, including liver fibrosis, cystic fibrosis and that of the lung, heart and kidney. Foxo3 transcription factor represses the Fibroblast Myofibroblast transition (FMT) which is involved in the pathogenesis of fibrosis. FOXO3 may also have beneficial effects on glucose metabolism through more favourable insulin sensitivity.FOXO3 controls cytokine production and lowers inflammation.

FOXO 3 transcription factor represses the Epithelial Mesenchymal transition (EMT) which is involved in the pathogenesis of fibrosis and in (EMT) cancers.

### Gene Expression

Experiments have shown that taltirelin modulates the gene expression in fibroblasts of a variety of genes involved in processes underlying ageing. Principally, the compound increases expression of the FOXO genes, notably FOXO1 and FOXO3.

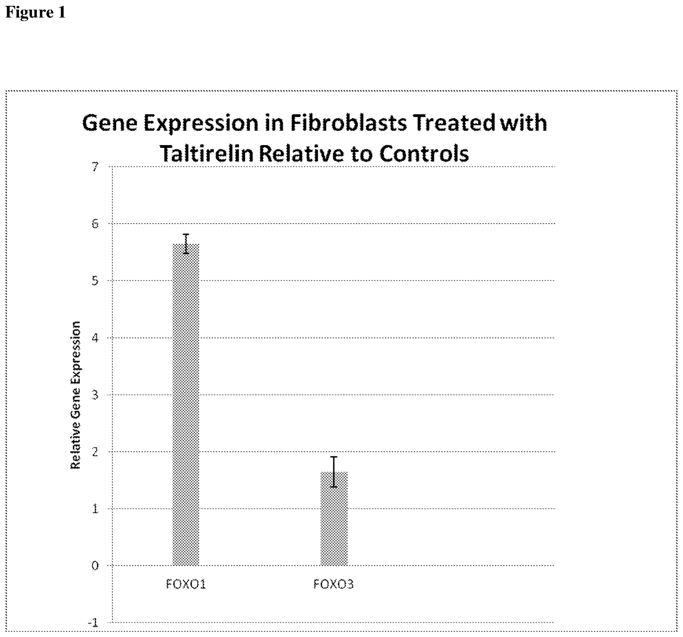

Figure 1 illustrates the increased expression of FOXO1 and FOXO3 genes that occurs in fibroblasts by administration of taltirelin. From the results, it can be seen that the administration of taltirelin in older cells (passage 20) led to a large increase in the expression of FOXO1 (>5X) and FOXO3 (>1.5X) compared to untreated controls. The results were statistically significant with P<0.01.

### COVID-19 caused by SARS-CoV-2

In certain people the immune response may be weak. The weakening of the immune response often occurs as people get older. In such people it would be advantageous to strengthen the immune response, for example, by expanding the T cell population.

FOXO 1 expands the population of T cells and B cells within an individual. T cells are produced by the thymus which decreases in size with age. The upregulation of FOXO 1 (as shown in Figure 1) has the effect of boosting the immune response, thus increasing the T cell population within an individual.

FOXO 1 also upregulates and enhances the survival of the CD8+ population that is involved in immune memory. For example, after an individual has had a viral attack their immune memory will result in an immediate response to a subsequent viral infection.

FOXO1 acts as the control point for a programme of gene expression that allows T-cells to undergo serial reactivation and renewal involved in long term immunity.

In addition, FOXO 3 upregulates and activate the interferon group (i.e. interferon α, interferon β and interferon γ). Interferon γ inhibits IL-13. IL-13 activates a receptor (TMPRSS2) on a host cell that severe acute respiratory syndrome coronavirus 2 (SARS-CoV-2) uses to enter the cell. FOXO 3 has been shown to inhibit the mechanism by which SARS-CoV-2 enters the host cell, via the TMPRSS2 receptor.

Advantageously, the composition of the present invention acts to block the activation of the TMPRSS2 receptor on host cells before and during a viral infection and to prevent cleavage of the spike protein of a coronavirus, particularly of SARS-CoV-2.

T-cells, including CD8+ T-cells are decreased markedly in patients with SARS-CoV-2 infection and there is functional exhaustion of cytotoxic lymphocytes associated with SARS-CoV-2 infection.

In addition, in adaptive immunity, FOXO1 increases expression of receptors that control T-cell trafficking to secondary lymphoid tissues and that the survival and homeostasis of T-cells are influenced by IL-7 and that FOXO1 controls T-cell tolerance and naive T-Cell homeostasis through induction of IL-7R expression.

Further, FOXO1 also regulates the homing of peripheral B cells through upregulation of L-selectin and regulates class-switch recombination in B-cells.

In addition, FOXO1 and FOXO3 are involved in oxidative stress resistance and that oxidative stress is a key player in viral infection including SARS-CoV-2 infection.

Thus, a composition comprising taltirelin or an analogue thereof may be used for the prevention, amelioration and/ or treatment of a condition caused by a virus such as a coronavirus, such as severe acute respiratory syndrome coronavirus 2 (SARS-CoV-2) via its effects of upregulating the expression of FOXO 1 and FOXO 3.

### The Sirtuins

SIRT1 (Sirt 1) expression was also increased significantly by taltirelin. Sirt 1 inhibits adipogenesis. Sirtion 1 expression induces autophagy. A high level of expression of SIRT1 is thought to be protective in many age-related diseases, including cardiovascular diseases, metabolic syndrome, neurodegeneration and cancer.

### The Ephrins

EFNB1 was significantly increased in fibroblasts by taltirelin. EFNB1 contributes to the suppression of the adipose inflammatory response and in obesity reduction on adipose EFNB 1 may contribute to adipose tissue inflammation.

### SERPINE1 (PAI-1)

SERPINE1 expression is significantly inhibited in fibroblasts by taltirelin. Obese patients with the age-related disease, metabolic syndrome and type-2 diabetes show an increase in levels of the product of this gene (PAI-1). Increased expression of this gene is also associated with enhanced radioresistance and tumour aggressiveness in non-small cell lung cancer cells. Furthermore, the product of the gene SERPINE1 (PAI-1) is the main inhibitor of the plasminogen activator system, which blocks fibrinolysis and promotes ECM accumulation in tissues therefore promoting fibrosis.

### NFE2L2 (Nrf2)

NFE2L2 expression is significantly increased in fibroblasts by taltirelin. The product of the gene NFE2L2 (Nrf2) inhibits EMT and fibrosis. NFE2L2 (Nrf2) expression decreases with age. NFE2L2 (Nrf2) expression is a critical regulator of the innate immune response. NFE2L2 (Nrf2) has a crucial role in viral entry and replication in tissues of the nasal passages.

NFE2L2 (Nrf2) controls cellular redox balance and the expression of a wide array of genes involved in immunity and inflammation, including antiviral actions. Nrf2 activity declines with age, making the elderly more susceptible to oxidative stress-mediated diseases, which include type 2 diabetes, chronic inflammation, and viral infections.

NFE2L2 (Nrf2) may also control viral susceptibility and replication.

NFE2L2 (Nrf2) activating compounds can downregulate ACE2 and TMPRSS2mRNA expression. ACE2 is a surface receptor and TMPRSS2 activates the spike protein for SARS-Cov-2 entry into host cells.

Further, NFE2L2 (Nrf2) is a master antioxidant transcription factor and is abundant in the lungs, drives diverse cellular defence pathways to counteract detrimental stimuli that are involved in the pathogenesis of these pulmonary disorders, including oxidative stress, inflammation, apoptosis, and carcinogenesis.

Thus, a composition comprising taltirelin or an analogue thereof may be used for the prevention, amelioration and/ or treatment of a condition caused by a virus such as a coronavirus, such as severe acute respiratory syndrome coronavirus 2 (SARS-CoV-2) via its effects on the innate immune response and on viral entry to the body and subsequent replication in tissues at the point of entry.

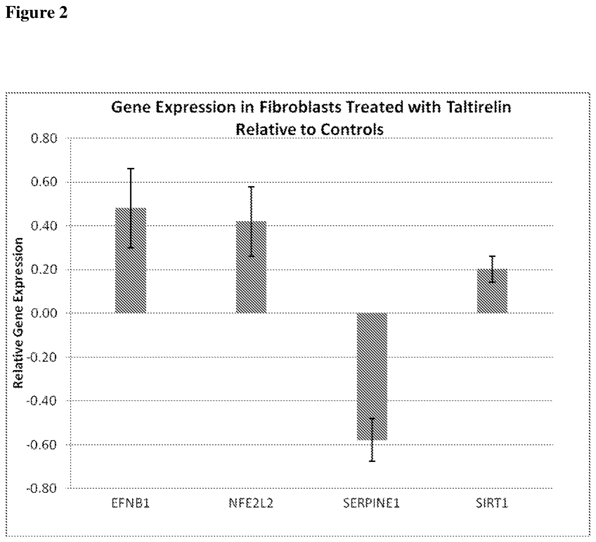

Figure 2 illustrates the increased expression of EFNB1, NFE2L2, SERPINE1 and SIRT1 genes in fibroblasts that occurs by administration of taltirelin. From the results, it can be seen that the administration of taltirelin in older cells (passage 20) led to a statistically significant (P<0.01) increase in the expression of EFNB1, NFE2L2 and SIRT1 genes and a statistically significant (P<0.01) decrease in the expression of the SERPINE1 gene compared to untreated controls.

Some further aspects of the invention can be found in the following paragraphs:
1. Use of a composition for the prevention, amelioration and/ or treatment of a condition associated with cell or tissue senescence and/ or inflammation and/ or autophagy, the composition comprising an active ingredient taltirelin (N-[[(4S)-Hexahydro-1-methyl-2,6-dioxo-4-pyrimidinyl]carbonyl]-L-histidyl-L-prolinamide) having the structure: 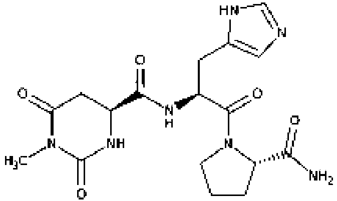 or an analogue thereof.
2. Use of the composition of paragraph 1, wherein the composition comprises anti-senescence, anti-inflammatory and/ or anti-proliferative and/ or pro-autophagic and/ or pro- stem cell regenerative properties and/ or stem cell pool maintenance properties.
3. Use of a composition according to paragraph 1 or 2 for the prevention, amelioration and/ or treatment of a condition caused by an infective agent.
4. Use of a composition according to any preceding paragraph, for the prevention, amelioration and/ or treatment of a condition caused by a virus.
5. Use of a composition according to paragraph 3 or 4, for the prevention, amelioration and/ or treatment of a condition caused by a coronavirus.
6. Use of a composition according to paragraph 3, 4 or 5, for the prevention, amelioration and/ or treatment of a condition caused by severe acute respiratory syndrome coronavirus 2 (SARS-CoV-2).
7. Use of a composition according to any of the proceeding paragraphs to augment the immune response, induce the clonal expansion of T-cell and B-cell populations, increase the reactivation of T-cells and increase the persistence of immune T-cell memory via the upregulation and enhanced survival of CD8+ cells.
8. Use of a composition according to any of the proceeding paragraphs to control T-cell trafficking to other tissues and to regulate the homing of peripheral B-cells.
9. Use of a composition according to any of the proceeding paragraphs for the prevention, amelioration and/ or treatment of a condition resulting from oxidative stress, in particular autoimmune diseases, diseases that are stress mediated and infection, including viral infection, including SARS-CoV-2.
10. Use of a composition according to any of the proceeding paragraphs to decrease fibrin deposition in the lungs so as to ameliorate lung pathology during and or after viral infection and to inhibit the activation of pathogenic Th17 cells to ameliorate their effect on lung pathology.
11. Use of a composition, according to any of the proceeding paragraphs to block the activation of the TMPRSS2 receptor on host cells before and during a viral infection and to prevent cleavage of the spike protein of a coronavirus, particularly of SARS-CoV-2.
12. Use of a composition, according to any of the proceeding paragraphs to increase the innate immune response including reducing susceptibility to viral infection, viral entry to patients and/ or viral replication.
13. Use of a composition according to any one of the preceding paragraphs for the prevention, amelioration and/ or treatment of an inflammatory and/ or autoimmune condition.
14. Use of a composition according to any one of the preceding paragraphs for the prevention, amelioration and/ or treatment of ulcerative colitis and/ or multiple sclerosis.
15. Use of the composition according to any preceding paragraph, for the prevention, amelioration and/ or treatment of a condition associated with fibrosis.
16. Use of the composition according to any one of the preceding paragraphs for the prevention, amelioration and/ or treatment of idiopathic renal fibrosis, fibrosis occurring in transplanted organs or tissues, idiopathic pulmonary fibrosis (the basis of COPD), cystic fibrosis and cardiac fibrosis.
17. Use of a composition according to any one of the preceding paragraphs for the prevention, amelioration and/ or treatment of a condition wherein fibrosis affects the subcutaneous fat of an individual.
18. Use of a composition according to any one of the preceding paragraphs for the prevention, amelioration and/ or treatment of a condition associated with metabolic syndrome.
19. Use of a composition according to paragraph 18, wherein the composition suppresses adipogenesis and regulates glucose metabolism.
20. Use of a composition according to paragraph 18 or 19, for the prevention, amelioration and/ or treatment of a condition associated with obesity, type 2 diabetes and associated disorders.
21. Use of a composition according to any one of the preceding paragraphs for the prevention, amelioration and/ or treatment of a cancer and/ or a condition associated with a drug resistant cancer.
22. Use of a composition according to any one of the preceding paragraphs for the prevention, amelioration and/ or treatment of a condition associated with epithelial to mesenchymal cancer (EMT cancer).
23. Use of a composition according to paragraph 22, for the prevention, amelioration and/ or treatment of epithelial cancers.
24. Use of a composition according to any one of paragraphs 21 to 23, for the prevention, amelioration and/ or treatment of cancer to include non small cell lung carcinoma, hepatocellular carcinoma, pancreatic ductal adenocarcinoma, cancer of the breast, cancer of the prostate, secondary brain tumours, and/ or primary brain tumours including glioblastoma.
25. Use of a composition according to any one of the preceding paragraphs for the prevention, amelioration and/ or treatment of a bone related disease.
26. Use of a composition according to paragraph 25, for the prevention, amelioration and/ or treatment of osteoarthritis, periodontitis, invertebral disk degeneration, osteosarcoma, osteoporosis and/ or cleidocranial dysplasia syndrome.
27. Use of a composition according to any one of the preceding paragraphs for the prevention, amelioration and/ or treatment of a skin condition.
28. Use of a composition according to paragraph 27 for the prevention, amelioration and/ or treatment of acne, psoriasis, ezcema and/ or vitiligo.
29. Use of a composition according to any one of the preceding paragraphs, wherein the composition is used to modulate and/ or counter the side effects of glucocorticoids.
30. Use of a composition according to paragraph 29, wherein the composition is synergistically used in combination with glucocorticoids.
31. Use of a composition according to paragraph 30, wherein the composition is used in parallel with glucocorticoids to reduce the risk of conditions such as osteoporosis and/ or skin thinning.
32. Use of a composition according to any one of the preceding paragraphs, for the prevention, amelioration and/ or treatment of inflammatory diseases.
33. Use of a composition according to paragraph 32 for the prevention, amelioration and/ or treatment of Crohn's disease, rheumatoid arthritis, lupus and/ or inflammatory bowel disease.
34. Use of a composition according to any one of the preceding paragraphs, for the promotion of neurogenesis.
35. Use of a composition according to any of the preceding paragraphs, for the prevention, amelioration and/ or treatment of cognition and/ or cognitive ability or poor memory in human subjects.
36. Use of a composition according to any one of the preceding paragraphs, for the prevention, amelioration and/ or treatment of diseases of the eye, including glaucoma, presbyopia and/ or macular degeneration.
37. Use of a composition according to any one of the preceding paragraphs, for the prevention, amelioration and/ or treatment of skeletal age-related muscle atrophy and/ or skeletal muscular dystrophy.
38. Use of a composition according to any one of the preceding paragraphs, for the prevention, amelioration and/ or treatment of malaria.
39. Use of a composition according to any one of the preceding paragraphs, for the promotion of longevity.
40. Use of a composition for the prevention, amelioration and/ or treatment of a condition associated with a virus, a condition associated with a coronavirus, a condition associated with severe acute respiratory syndrome coronavirus 2 (SARS-CoV-2), a condition associated with fibrosis such as idiopathic renal fibrosis, fibrosis in transplanted tissues and organs, idiopathic pulmonary fibrosis (the basis of chronic obstructive pulmonary disease, i.e. COPD), cystic fibrosis; a condition associated with reduced cellular autophagy; a condition associated with a reduction in stem cell pool maintenance and/ or stem cell regenerative capability; a condition associated with metabolic syndrome; a condition associated with obesity, type 2 diabetes and associated disorders; a condition associated with cancer, such as a drug resistant cancer, epithelial to mesenchymal (EMT) cancer, epithelial cancer, bone cancer, non small cell lung carcinoma, hepatocellular carcinoma, pancreatic ductal adenocarcinoma, glioblastoma, brain primary cancers and metastases; a bone related condition such as osteoarthritis, periodontitis, invertebral disk degeneration, osteosarcoma, osteoporosis, cleidocranial dysplasia syndrome; a skin condition such as acne, psoriasis, eczema, vitiligo; and/ or malaria, the composition comprising an active ingredient taltirelin (N-[[(4S)-Hexahydro-1-methyl-2,6-dioxo-4-pyrimidinyl]carbonyl]-L-histidyl-L-prolinamide) having the structure: 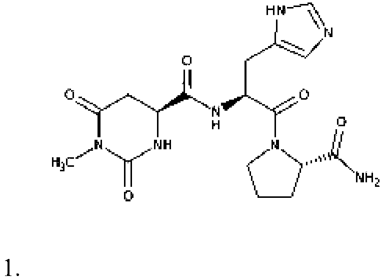 or an analogue thereof.

## Claims

1. Taltirelin (N-[[(4S)-Hexahydro-1-methyl-2,6-dioxo-4-pyrimidinyl]carbonyl]-L-
histidyl-L-prolinamide) having the structure: 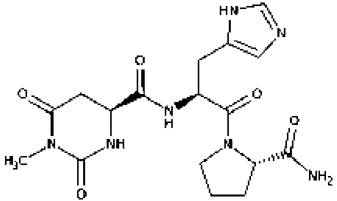 or an analogue thereof,
for use in the treatment of a condition associated with cell or tissue senescence or inflammation, wherein the condition is periodontitis, invertebral disk generation, osteosarcoma, osteoporosis, cleidocranial dysplasia syndrome, rheumatoid arthritis, lupus, Crohn's disease, inflammatory bowel disease, ulcerative colitis, multiple sclerosis, fibrosis, type II diabetes, metabolic syndrome or for use in the promotion of neurogenesis.

2. Taltirelin according to claim 1 for the treatment of idiopathic renal fibrosis, fibrosis occurring in transplanted organs or tissues, idiopathic pulmonary fibrosis (the basis of COPD), cystic fibrosis or cardiac fibrosis.
